# FASCICULE DE BREVET EUROPEEN

(11) **EP 1 165 582 B1**
(45) Date de publication et mention de la délivrance du brevet: **15.01.2003**
(21) Numéro de dépôt: 00915231.5
(22) Date de dépôt: 29.03.2000
(51) Int. Cl.: C07H 17/04, C07H 13/04

(54) **NOUVEAU PROCEDE DE PREPARATION DE L'ETOPOSIDE**
NEUES VERFAHREN ZUR HERSTELLUNG VON ETOPOSID
NOVEL METHOD FOR THE PRODUCTION OF ETOPOSIDE

(30) Priorité: 29.03.1999 FR 9903876
(43) Date de publication de la demande: 02.01.2002
(73) Titulaire: PIERRE FABRE MEDICAMENT, 92100 Boulogne (FR)
(72) Inventeur: GUMINSKI, Yves, F-81090 Lagarrigue (FR); IMBERT, Thierry, F-81290 Viviers les Montagnes (FR); FABRE, Jean, F-81500 Lavaur (FR)
(74) Mandataire: Ahner, Francis
(86) Numéro de dépôt international: FR0000774
(87) Numéro de publication internationale: WO00058326

(56) Documents cités:
- EP-A- 0 445 021
- WO-A-93/02094

## Description

La présente invention a pour objet un nouveau procédé de préparation de l'étoposide. Il consiste à préparer le 1-O-triéthylsilyl-2,3-diphénoxyacétyl-4,6-éthylidène-β-D-glucoside et de le condenser au 4'-phénoxyacétyl-4'-déméthyl épipodophyllotoxine. Ce procédé a l'avantage d'utiliser un intermédiaire stable et cristallin et de s'affranchir de la présence d'isomère α-glucoside et des sous-produits pour fournir ainsi l'étoposide par un procédé facilement industrialisable.

L'étoposide de formule 1 est un composé hémisynthétique utilisé dans le traitement du cancer en particulier les carcinomes embryonnaires du testicule, les sarcomes conjonctifs, les tumeurs pédiatriques, les cancers bronchiques à petites cellules, les lymphomes malins, hodgkiniens et non hodgkiniens, les leucémies aiguës, les choriocarcinomes placentaires, les adénomes carcinomes mammaires, les cancers colorectaux et les cancers du poumon non à petites cellules.

L'étoposide est formé d'une partie glycosidique, en particulier le 4,6-éthylidène-β-D glucopyranose et d'une partie lignane la 4'-déméthyl épipodophyllotoxine.

Ce composé hémisynthétique a été décrit et breveté dans CH 514578 et US-A-3524844. Il a fait l'objet de plusieurs publications, en particulier J. Med. Chem (1971) 14, 936 ; JOC (1981), 46, 2826 ; Heterocycles (1991), 32, 859 ; Tetrahedron (1991), 47, 4675 ; Synthesis (1991), 275 ; J.Org.Chem. (1993), 58, 4175 ; Tet.Let. (1992), 33, 4831.

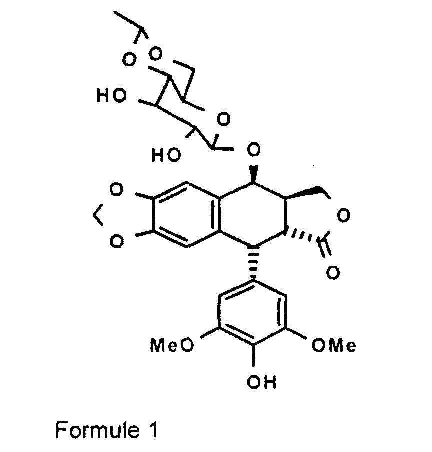

Le problème posé par la synthèse de l'étoposide est entre autres celui de la pureté anomérique de la jonction glycosidique.

De la pureté anomérique du glucopyranose de départ pour le couplage, dépendra la pureté anomérique de l'étoposide en fin de synthèse.

La purification de l'étoposide pour séparer les 2 anomères α et β est extrêmement difficile, d'un point de vue industriel et nécessite un traitement chromatographique coûteux, les 2 composés ayant des temps de rétention en CLHP très voisins.

La Demanderesse a breveté une préparation de l'étoposide (EP 445 021), faisant intervenir le 1-O-carbobenzyloxy-2,3-bisphénoxyacétyl-4,6-éthylidène-β-D-glucopyranoside, lequel après hydrogénolyse et par couplage avec le 4'-phénoxyacétyl-4'-démethylépipodophyllotoxine fournissait, après déprotection des groupements phénoxyacétyls, l'étoposide avec une excellente pureté. Cependant, cette méthode nécessitait l'utilisation d'une étape d'hydrogénolyse catalytique sur métaux précieux tels le palladium, ce qui élevait le coût de la synthèse. De plus, l'intermédiaire alcool anomère, issu de cette hydrogénolyse, bien que de stabilité suffisante, devait être engagé sans trop attendre dans l'étape de couplage, pour éviter une anomérisation, qui conduirait inévitablement à la présence de l'anomère α.

La Demanderesse a montré l'intérêt d'utiliser des groupements phénoxyacétyle sur les positions 2 et 3 de l'éthylidène glucose. Ces groupements protecteurs donnent des produits plus stables, au lieu des groupements plus classiques tels que acétyl ou chloro, dichloro acétyl. Ces groupements se sont avérés de meilleurs stabilisants au regard du risque d'anomérisation. De plus, ces groupements phénoxyacétyle se clivent dans des conditions relativement douces compatibles avec les autres fonctions chimiques de la molécule entière, par exemple, par traitement avec l'acétate de Zinc ou bien encore en présence d'une amine (voir Protective Groups in Organic Synthesis : Th. Greene , Ed : Wiley and Sons 2^{nd} edition 1991, p 92 et 96).

La silylation des alcools anomères des sucres est bien connue pour fournir des alcools anomères silylés en position β exclusivement. Voir Chem. Ber. (1964), 97, 2196, où les auteurs préparent le trimethylsilyl tétra acétyl glucose en tant qu'anomère β pur. Ceci a été repris également par Tietze (Synthesis (1982), 946). De même, Tet. Let (1991) 32, 2079 indique la préparation de sucres silylés en position anomérique sous forme d'anomère β de façon prédominante. Ces sucres silylés ont été utilisés pour des réactions de glycosylation en présence de triméthylsilyltriflate ou d'éthérate de BF₃ par exemple dans Tet. Let. (1982), 23, 4661, ainsi que dans Synth. Comm. (1989), 19, 3453.

Une préparation de l'étoposide a récemment été également brevetée, WO.93/02094 correspondante aux publications Tet.Let. (1992) 33, 4831, et J.Org.Chem. (1993) 58, 4175 où il est fait état de la condensation d'un glucopyranoside silylé avec la 4'-déméthyl épipodophyllotoxine non protégée en position 4', qui évitait l'étape de protection du phénol en position 4'. Cette méthode apportait une amélioration du procédé, mais utilisait un intermédiaire 4,6-éthylidène-2,3-chloro ou dichoroacétyl-1-O-triméthylsilyl-β-D-glucopyranoside peu stable, au niveau du groupement triméthylsilyloxy anomérique.

De plus, la réaction de glycosylation sur la 4'-déméthylépipodophyllotoxine non protégée en position 4', est susceptible d'engendrer des sous-produits d'autocondensation, difficilement éliminables par la suite.

La présente invention concerne un procédé de préparation de l'étoposide consistant en la préparation de l'intermédiaire 2,3-diphénoxyacétyl-4,6-éthylidène-1-O-triéthylsilyl-β-D-glucopyranoside, de formule 2 pour être condensé en présence d'un acide de Lewis, sur la 4'-phénoxyacétyl-4'-déméthyl épipodophyllotoxine, et permet ainsi de répondre à la problématique.

L'intermédiaire de formule 2 possède le groupe triéthylsilyloxy en position β équatoriale uniquement.

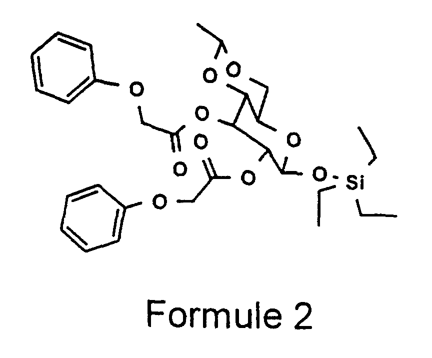

C'est un composé stable, cristallin, recristallisable donc bien purifiable et conservable dans des conditions usuelles, sans précautions particulières. La poudre obtenue peut être stockée à la température ambiante du laboratoire ou de l'atelier pendant quelques mois ou plus sans altération. Les rendements de sa préparation sont satisfaisants, et le procédé est aisé à mettre en oeuvre.

La préparation du composé glucoside, de formule 2, se fait à partir de l'éthylidène glucose par acylation des 3 fonctions alcools par le chlorure d'acide de l'acide phénoxyacétique dans le chlorure de méthylène et la pyridine. L'hydrolyse des phénoxyacétyle, en mélange, en position anomérique s'effectue en présence d'une amine, telle que l'éthanolamine. On obtient à ce stade le mélange des deux alcools anomères. La silylation de ce mélange par le chlorure de triéthylsilyle en présence de triéthylamine dans le chlorure de méthylène, fournit ainsi l'intermédiaire de formule 2 en tant qu'anomère β pur.

L'utilisation du groupement triéthylsilyle est important et particulier. Il est trouvé de façon remarquable la grande stabilité de l'intermédiaire de formule 2 par rapport à son analogue triméthyl silylé. En effet, le dérivé 1-β-O-triméthylsilyle est moins cristallin. Il ne se conserve pas et se trouve instable en présence de silice, dans les conditions du suivi des réactions par ccm. Il ne peut donc pas être utilisé dans ces conditions. Ce phénomène est essentiel d'un point de vue industriel quand l'opération s'effectue sur des quantités de l'ordre de quelques kilos ou plus, de produit à manipuler et à transformer.

La condensation de cet intermédiaire de formule 2 avec la 4'-phénoxyacétyl-4'-déméthyl épipodophyllotoxine s'effectue en présence d'éthérate de BF₃ dans un solvant inerte comme le chlorure de méthylène à une température entre -15°C à -25°C. On obtient à ce stade le 2",3",4'-triphénoxyacétyl de l'étoposide. Ce composé a déjà été obtenu dans notre précédent brevet (Fr 2 658 824). Après déprotection des groupements phénoxyacétyls par l'éthanolamine dans le chlorure de méthylène et le méthanol ou l'acétate de Zinc dans le méthanol additionné de THF, on obtient l'étoposide de qualité conforme.

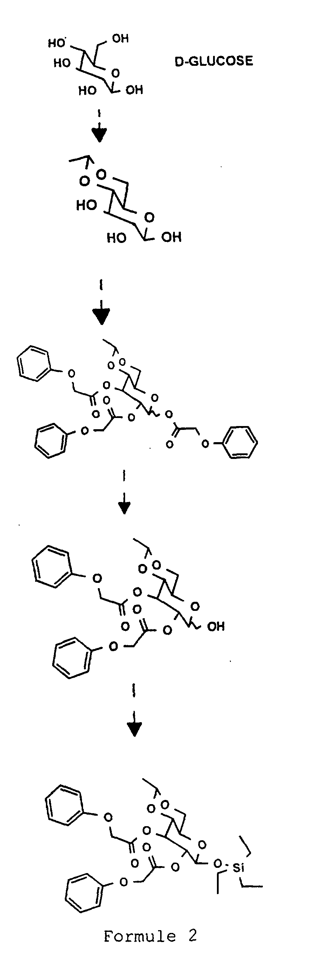

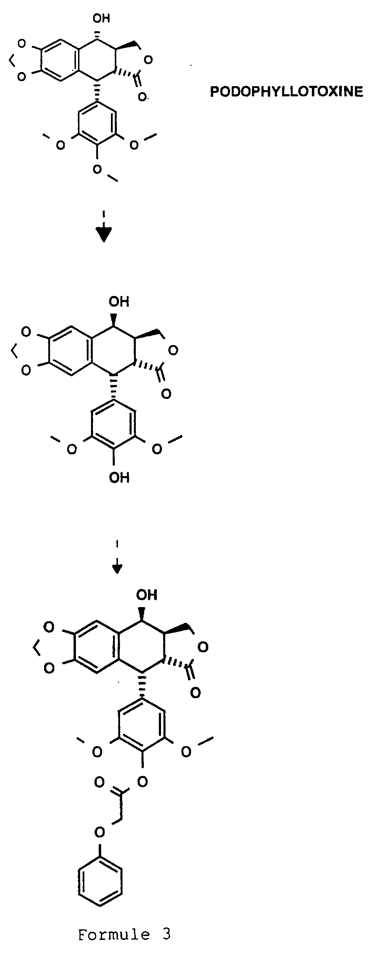

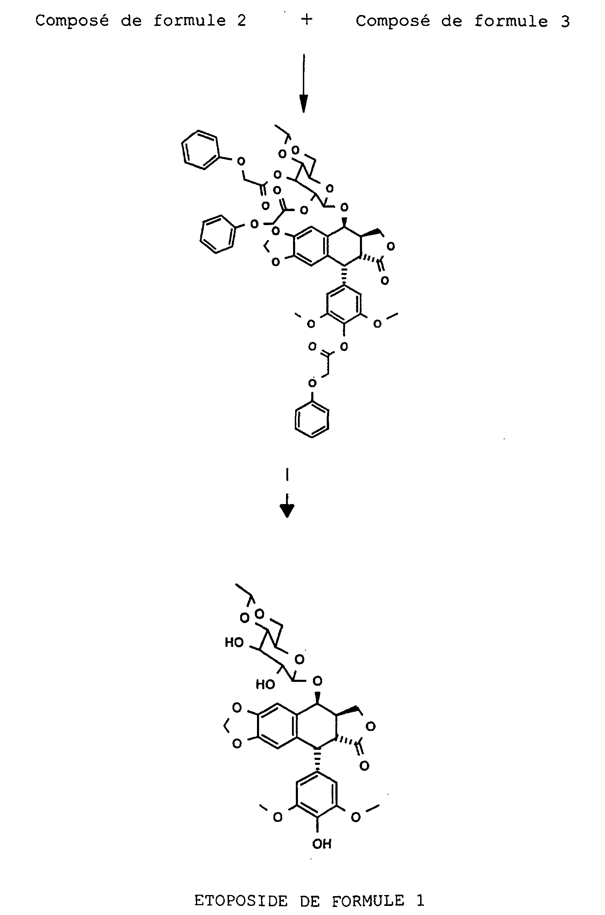

Le schéma général de synthèse de l'étoposide est illustré à la figure 1 annexée.

Les modes opératoires suivants permettent d'illustrer l'intérêt de la présente invention.

### Etape 1 : 1,2,3-Triphénoxyacétyl-4,6-éthylidène glucose

100 kg d'ethylidène glucose sont dissous dans 1 000 L de chlorure de méthylène sous agitation. 257 kg de chlorure de l'acide phénoxyacétique sont alors coulés dans le milieu réactionnel à un rythme pour garder la température du réacteur à 15°C. Après que la réaction soit complète, le milieu réactionnel est lavé successivement avec 1000 L d'HCI 2N, 1000 L d'eau, puis avec 1000 L d'une solution de bicarbonate de Na à 8 %, et enfin avec 1000 L d'eau. La phase organique est additionnée de toluène et évaporée en conservant un volume de 550 L consistant en triester brut dans le toluène. Cette solution est utilisée directement dans l'étape suivante.

### Etape 2 : 2,3-diphénoxyacétyl-4,6-éthylidène glucose

On additionne à la solution toluénique de l'étape 1, un mélange de 275 L de dioxane et 275 L de toluène, puis on refroidit cette solution à -10°C. On additionne alors 35,5 L d'éthanolamine, en solution dans 275 L de méthanol, lentement au milieu réactionnel en maintenant la température à -10°C. L'agitation est maintenue pendant 6 h, l'avancée de la réaction est suivie par ccm. Le milieu réactionnel est ensuite lavé et décanté successivement par 700 L d'eau, par 3 fois 700 L d'HCI N, par 700 L d'eau avant de neutraliser avec 3 fois 700 L d'une solution de bicarbonate de sodium à 8 %. Après un nouveau lavage avec 700 L d'eau, la phase organique est concentrée sous vide sans venir à sec (solution encore agitable), puis on y additionne 275 I de chlorure de méthylène. Cette solution est utilisée directement dans l'étape suivante.

### Etape 3 : 2,3-diphénoxyacétyl-4,6-éthylidène-1-O-triéthylsilyl-β-D-glucopyranoside

A la solution du diester dans le chlorure de méthylène obtenue au stade précédent, sont additionnés 136 L de triéthylamine. La solution ainsi obtenue est refroidie à 0°C. On ajoute alors lentement 133 L de chlorure de triéthylsilane en prenant soin de ne pas dépasser + 5°C dans la solution réactionnelle. A la fin de l'addition, l'agitation est maintenue pendant 3 h et l'avancée de la réaction est suivie en ccm. Ensuite, le milieu réactionnel est lavé et décanté successivement par 500 L d'une solution aqueuse de bicarbonate de Na à 8 %. La phase organique est filtrée, évaporée sous vide à une température ≤ 50°C. En fin d'évaporation, on rajoute de l'éther isopropylique. Le dérivé silylé diester cristallise et est récupéré par filtration. Après séchage à 60°C sous vide à l'étuve pendant 48 H, on obtient 128 kg de dérivé 2,3-diphénoxyacétyl-4,6-ethylidène-1-O-triethylsilyl-β-D-glucopyranoside, ce qui représente un rendement de 42 % à partir de l'éthylidène glucose (F°C =120°C). Ce composé se conserve très bien. Chromatographie sur couche mince de silice: solvant : heptane- acetate d'éthyle 50/50 (Rf 0.75).

¹H RMN, 400 MHz, (CDCl₃): d = 0.59 (q, 6H, J = 8Hz, Si-C*H*_{*2*}), 0.93 (t, 9H, J = 8Hz, SiCH₂C*H*_{*3*}), 1.32 (d, 3H, J = 4.8Hz, H-8), 3.39 (m, 1H, H-6ax), 3.51 (t,1H, J = 9.6 Hz, H-4), 3.57 (t, 1H, H-5, J = 10.4 Hz), 4.15 (dd, 1H, H- 6eq), 4.55 (d, 2H, J = 4 Hz, OC*H*_{*2*}CO), 4.58 (d, 2H, J = 4 Hz, OC*H*_{*2*}CO), 4.66 (m, 1H, H₇), 4.84 (d, 1H, H₁, J₁₋₂=7.6 Hz), 5.01 (t, 1H, J = 7.6 Hz, H-2), 5.30 (t, 1H, J = 9.3 Hz, H-3), 6.88 (m, 4H_{arom.ortho}), 6.96 (t, 2H_{arom.para}, J = 7.4 Hz), 7.26 (m, 4H_{arom.meta}).

### Etape 4 : 2",3"-bisphénoxyacétyl-4",6"-éthylidène-β-D-glucoside de 4'-phénoxy-acétyl-4'-déméthylépipodophyllotoxine.

8,4 kg (15,7 moles) de 4'-phénoxyacétyl-4'-déméthyl épipodophyllotoxine sont dissous dans 30 L de chlorure de méthylène, puis on y introduit 12 kg (22,5 moles) de dérivé silylé, obtenu au stade précédent, dissous dans 36 L de chlorure de méthylène. La solution est refroidie à -25°C sous agitation, puis on introduit alors 6,7 kg (47,2 moles) d'éthérate de trifluorure de bore, sous atmosphère inerte, où le milieu est maintenu pendant 9 h. Le suivi de la réaction est observé par ccm. L'hydrolyse du milieu s'effectue par de l'eau (2 fois 80 L). La phase organique est de nouveau lavée par 80 L d'eau, séchée sur Na₂SO₄, et concentrée jusqu'à un volume de 60 L. Cette solution est engagée directement dans l'étape suivante.

### Etape 5 : Etoposide

Au composé brut précédent en solution (60 L) sont ajoutés 60 L de méthanol. Sous agitation à -10°C, on introduit 8 kg d'éthanolamine au cours du temps en 4 fois toutes les heures. La réaction s'effectue en 6 h. L'avancement de la réaction est suivi par ccm. Le milieu réactionnel, après retour à température ordinaire, est acidifié à pH6 avec une solution d'HCl 1N (100 L), puis lavé par 2 fois 120 L d'eau. La phase organique est séparée, concentrée en partie, puis reprise à l'acétate d'éthyle (160 L) évaporée, puis reprise à nouveau par 80 L d'acétate d'éthyle. L'étoposide est obtenu précipité par refroidissement à 0°C sous agitation lente.

Le précipité est essoré, séché, pour conduire à 8,25 kg d'étoposide brut, qui recristallisé dans du méthanol et de l'éthanol aqueux conduit à de l'étoposide conforme en tous points à un échantillon authentique, et conforme aux exigences de la pharmacopée européenne et USP.

La même transformation peut s'effectuer à partir du produit de l'étape 4, après avoir évaporé le chlorure de méthylène, par un traitement avec l'acétate de Zinc dans du méthanol et du THF au reflux 5h, selon la méthode utilisée dans notre brevet Fr 2 658 824. Ce traitement conduit à l'étoposide avec un rendement de 75%. F°C=257-266°C
¹H RMN, 400 MHz : d = 1.24 (d, 3H, H-8"), 2.88 (m, 1H, H-3, J₃₋₄ = 3.6 Hz, J₃₋₁₁ = 8.4 Hz), 3.06 (m, 1H, H-2"), 3.16 (t, 1H, H-4"), 3.25 (m, 1H, H-5"), 3.27 (dd, 1H, H-2, J₂₋₃ = 14Hz), 3.34 (m, 1H, H-3"), 3.51 (t, 1H, H-6"ax), 3.61 (s, 6H, 3',5'-OCH₃), 4.08 (dd, 1H, H-6"eq), 4.26 (m, 2H, H-11a, H-11b), 4.49 (d, 1H, H-1, J₁₋₂ = 5.2Hz), 4.58 (d, 1H, H-1", J_{1"-2"} = 7.5Hz), 4.73 (q, 1H, H-7"), 4.93 (d, 1H, H-4), 5.26 (d, 2H, OH-2",3"), 6.02 (s,1H, H-15a), 6.04 (s, 1H, H-15b), 6.18 (s, 2H, H-2',6'), 6.53 (s, H-8), 7.01 (s, 1H, H-5), 8.28 ( s, 1H, OH-4').

## Revendications

1. Procédé de préparation de l'étoposide de formule 1 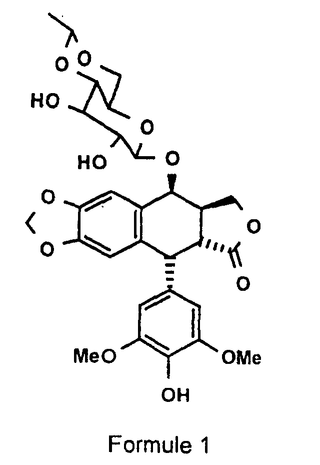 **caractérisé en ce qu'**il implique la condensation du 2,3-diphénoxyacétyl-1-O-triéthylsilyl-β-D-glucopyranoside de formule 2 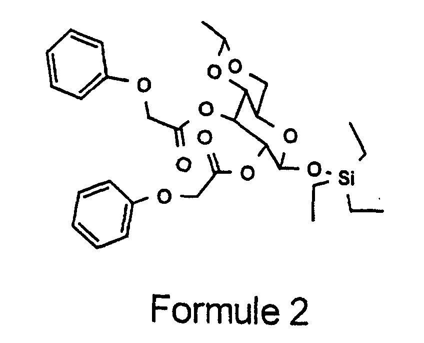 avec la 4'-phénoxyacétyl-4'-déméthylépipodophyllotoxine de formule 3

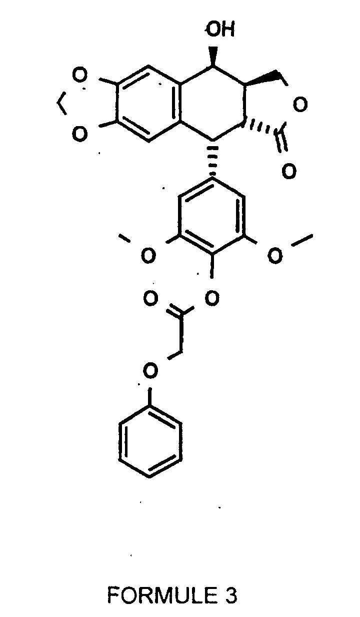

2. Composé de formule 2 ci-dessous, **caractérisé en ce que** la position anomère est sous forme exclusivement β

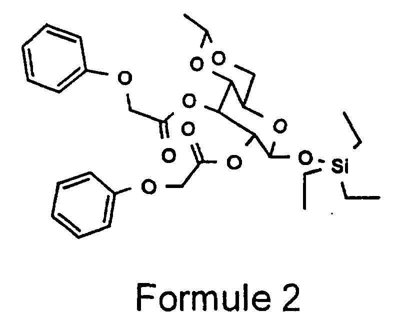

3. Procédé de préparation d'un composé de formule 2 selon la revendication 2, **caractérisé en ce que** l'on forme exclusivement l'anomère β à partir du 2,3-diphénoxyacétyl-4,6-éthylène-D-glucopyranose sous forme du mélange des deux alcools anomères, par réaction avec le chlorure de triéthylsilyle.

## Patentansprüche

1. Verfahren zur Herstellung von Etoposid der Formel 1 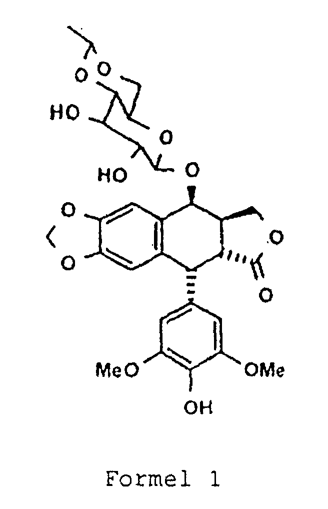 **dadurch gekennzeichnet, dass** es die Kondensation von 2,3-Diphenoxyacetyl-1-O-triethylsilyl-β-D-glucopyranosid der Formel 2 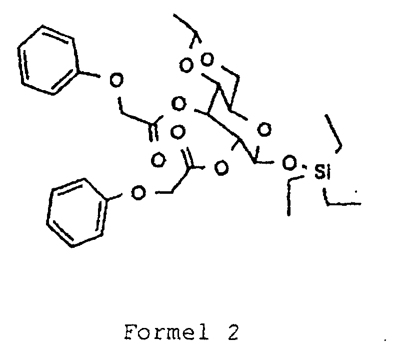 mit 4'-Phenoxyacetyl-4'-demethylepipodophyllotoxin der Formel 3 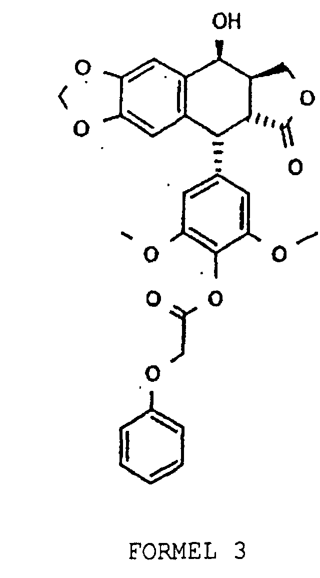 umfasst.

2. Verbindung der nachstehenden Formel 2, **dadurch gekennzeichnet, dass** die Anomerieposition ausschließlich in β-Form vorliegt

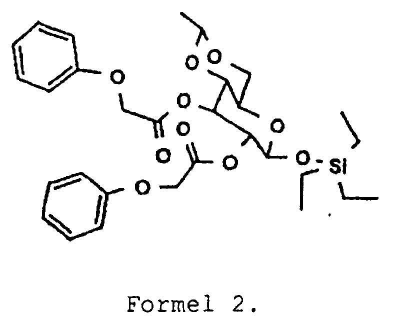

3. Verfahren zur Herstellung einer Verbindung der Formel 2 nach Anspruch 2, **dadurch gekennzeichnet, dass** man ausgehend von 2,3-Diphenoxyacetyl-4,6-ethyliden-D-glucopyranose in Form der Mischung der zwei anomeren Alkohole durch Umsetzung mit Triethylsilylchlorid ausschließlich das β-Anomer bildet.

## Claims

1. Process for the preparation of etoposide of formula 1 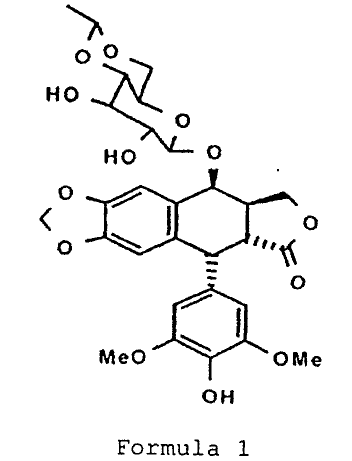 **characterized in that** it involves the condensation of the 2,3-diphenoxyacetyl-1-O-triethylsilyl-β-D-glucopyranoside of formula 2 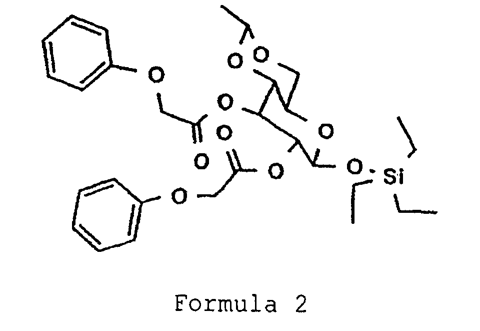 with 4'-phenoxyacetyl-4'-demethylepipodophyllotoxin of formula 3

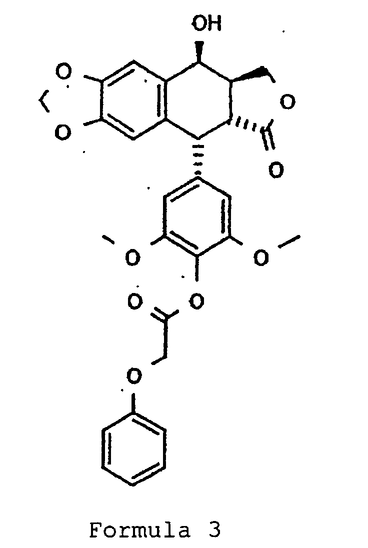

2. Compound of formula 2 below, **characterized in that** the anomeric position is exclusively in the β form

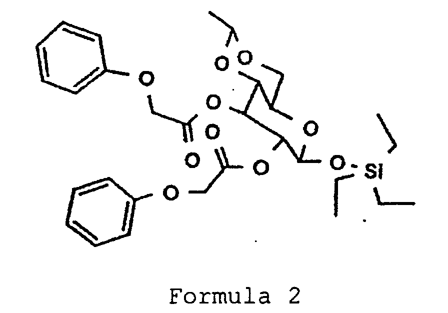

3. Process for the preparation of a compound of formula 2 according to Claim 2, **characterized in that** the β anomer is formed exclusively from 2,3-diphenoxyacetyl-4,6-ethylidene-D-glucopyranose, in the form of the mixture of the two anomeric alcohols, by reaction with triethylsilyl chloride.
